(19) Europäisches Patentamt
European Patent Office
Office européen des brevets

(11) EP 3 632 530 A1

(12) EUROPEAN PATENT APPLICATION

(43) Date of publication:
08.04.2020 Bulletin 2020/15

(21) Application number: 18197885.9

(22) Date of filing: 01.10.2018

(51) Int Cl.:
*B01J 8/02* (2006.01) *B01J 8/00* (2006.01)
*B01J 21/04* (2006.01) *B01J 23/46* (2006.01)
*B01J 23/75* (2006.01) *B01J 23/755* (2006.01)
*C10L 3/08* (2006.01) *C07C 1/12* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Ecole Polytechnique Fédérale de Lausanne (EPFL)**
  **1015 Lausanne (CH)**
- **Gaznat SA**
  **1003 Lausanne (CH)**

(72) Inventors:
- **Züttel, Andreas**
  **1950 Sion (CH)**
- **Gallandat, Noris**
  **1950 Sion (CH)**

(74) Representative: **reuteler & cie SA**
**Chemin de la Vuarpillière 29**
**1260 Nyon (CH)**

# (54) METHANATION REACTOR

(57) The present relates to a chemical reactor comprising a catalyst bed enclosed in a reactor vessel and at least one cooling tube placed in the reactor vessel and passing through the catalyst bed, characterized in that the cooling tubes are disposed within the reactor so as to generate thermal gradients of at least 20°C/cm thereby generating hot spots throughout the reactor upon carrying out a reaction.

Figure 1

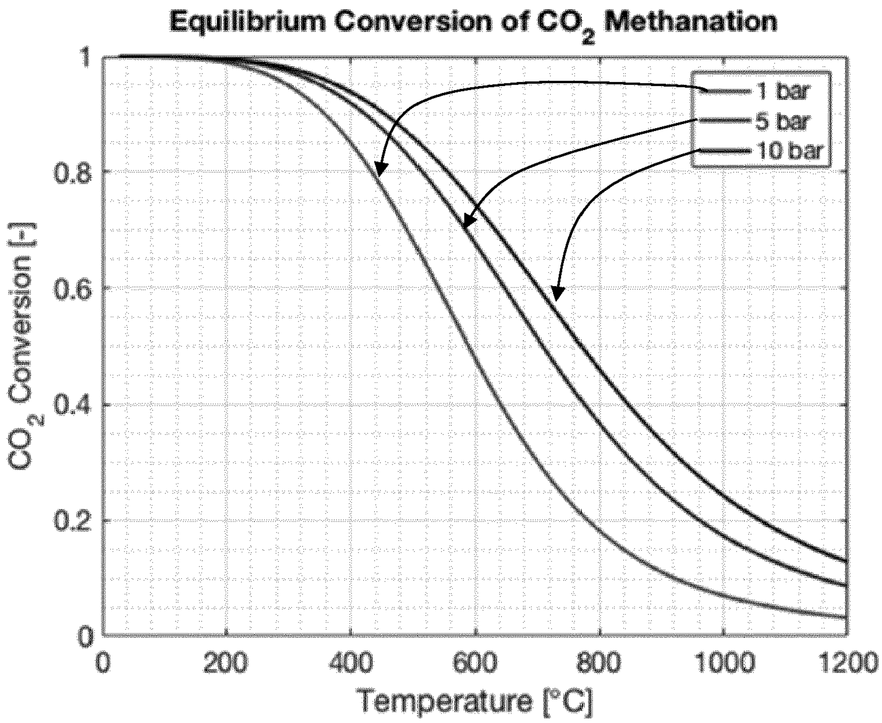


EP 3 632 530 A1

## Description

### Technical Field

The present invention relates to a chemical reactor and a thermal management system maximizing the conversion of the Sabatier reaction.

### Background of the art

The chemical reactions and processes that allow producing synthetic hydrocarbons from hydrogen and carbon dioxide have the potential to play an important role in the energy turnaround. The Sabatier reaction is especially interesting as it produces methane with a high selectivity, which can be directly integrated in the existing natural gas infrastructure.

The methanation reaction was first discovered by the French chemist Paul Sabatier at the beginning of the 20th century. The reaction describes the production of methane and water from hydrogen and carbon dioxide, as shown in equation ((1) below:

$$\begin{gathered} CO_2 + 4H_2 \leftrightarrow CH_4 + 2H_2O_{(l)} \\ \Delta H_R^0 = -252.8\ kJ/mol \\ \Delta S_R^0 = -409.9\ J/mol - K \end{gathered} \qquad (1)$$

As shown, this process is highly exothermic, with a reaction enthalpy of -252.8 kJ/mol at standard conditions (T = 273.15K, p = 1.013·105 Pa). Because of this, the thermodynamic equilibrium conversion is shifted to the reactant side at higher temperature. Figure 1 shows the maximal equilibrium conversion as a function of the temperature and at different pressures, and one can see that high conversion rates can only be achieved at low temperatures.

On the other hand, the kinetics of the reaction follow the Arrhenius law which means that the rate of reaction increases exponentially with the temperature, as shown in equation ((2) below and in figure 2.

$$k_f = k_0 e^{\left(-\frac{E_a}{RT}\right)} \qquad (2)$$

Thus, there is a problem arising in this chemical reaction that two competing trends have to be balanced: a higher temperature enables a faster reaction, but the thermodynamic conversion is limited, while a lower temperature limits the reaction rate but allows for a high thermodynamic equilibrium conversion.

In order to solve this technical problem, different types of reactor have been designed to improve the reaction yield. Today, there basically exist two main reactor designs: tube reactors and plate reactors. In general, both cases emphasize on how to minimize the temperature gradients within the reactor in order to reach an isothermal operation.

For example, document DE102014010055A1 discloses a methanation reactor comprising a two-phase cooling system and a process to control the temperature of the system and remove the heat of reaction by boiling a cooling medium. However, this document is silent regarding any reference to maintaining a specific temperature profile within the reactor in order to maximize the conversion.

Another document, DE102014011274 presents a heat exchanger design for a chemical reactor, and especially for a methanation reactor, that consists of plates welded together, and a circulating cooling medium in the space between the plates. A thermal coupling between the cooling medium and the gas products is thereby achieved and the temperature can be maintained accurately but thereis no mention of maintaining a specific temperature profile within the reactor in order to maximize the conversion.

In view of the above, a primary object of the invention is to solve the above-mentioned problems and more particularly to provide a methanation reactor designed and adapted to provide and maintain a specific temperature profile within the reactor in order to maximize the Sabatier conversion, or any other exothermic reaction.

### Summary of the invention

The above problems are solved by the present invention.

A first aspect of the invention is a chemical reactor comprising a catalyst bed enclosed in a reactor vessel and at least one cooling tube placed in the reactor vessel and passing through the catalyst bed, characterized in that the cooling tubes are disposed within the reactor so as to generate thermal gradients of at least 20°C/cm thereby generating hot spots throughout the reactor upon carrying out a reaction.

According to a preferred embodiment of the present invention, the catalysts comprises at least one of Nickel, Cobalt and Ruthenium based catalysts.

Advantageously, the catalysts comprises 20% wt. $Ni/Al_2O_3$ or 3% wt. $Ru/Al_2O_3$.

Preferably, the chemical reactor comprises at least two cooling tubes.

According to a preferred embodiment of the present invention, the minimal distance between the tubes is not less than 1.5 times the tube diameter.

Advantageously, the minimal distance between the tubes is not less than 2 times the tube diameter.

Preferably, the temperature of the cooling medium is different in the different tubes.

According to a preferred embodiment of the present invention, the tubes are fed with a cooling medium such as water, oil or any other fluid suitable for this purpose.

Advantageously, the thermal gradients is at least 100°C/cm

Preferably, the temperature gradients are controlled by controlling either the space velocity of the inlet reactant gases and/or the flow rate of the cooling medium.

According to a preferred embodiment of the present invention, the chemical reactor comprises a thermal management system adapted to remove the heat from the reaction zone and to control the temperature of the chemical reactor.

According to a preferred embodiment of the present invention, the reactor is adapted for an exothermic chemical reaction.

The particular advantages of this device of the invention are to solve the technical problem identified above..

## Brief description of the drawings

Further particular advantages and features of the invention will become more apparent from the following non-limitative description of at least one embodiment of the invention which will refer to the accompanying drawings, wherein

- Figure 1 represents the Thermodynamic equilibrium conversion as a function of the temperature at different pressures;
- Figure 2 represents the kinetics of CO2 methanation as a function of the temperature;
- Figure 3 schematically represents a first embodiment of the methanation reactor;
- Figure 4 schematically represents an experimental $CO_2$ conversion at different temperatures and space velocities;
- Figure 5 schematically represents the experimental setup for the methanation reactor;
- Figure 6 schematically represents a calculated temperature distribution in the cross-section of the methanation reactor (FEA Simulation).

## Detailed description of the invention

The present detailed description is intended to illustrate the invention in a non-limitative manner since any feature of an embodiment may be combined with any other feature of a different embodiment in an advantageous manner.

Figure 3 shows a first embodiment of the methanation reactor of the present invention which is a chemical reactor for exothermic chemical reaction occurring in the gas phase on a heterogeneous catalyst. The cooling system of the reactor is arranged such that high thermal gradients are present within the reactor, favoring high equilibrium conversion and fast reaction rate simultaneously.

As one can see, the reactor comprises a catalyst bed enclosed in a reactor vessel. The catalysts preferably comprises at least one of Nickel, Cobalt and Ruthenium based catalysts, for example 20% wt. $Ni/Al_2O_3$ or 3% wt. $Ru/Al_2O_3$.

It also comprises one or several cooling tubes placed in the reactor vessel and passing through the catalyst bed and which are fed with a cooling medium such as water, oil or any other fluid suitable for this purpose. Preferably the cooling medium is at ambient Temperature when fed in, but this is clearly not mandatory and it is possible to have an homogeneous coolant temperature in the tubes or a serial arrangement where the temperature would be different in the different tubes.

The cooling tubes are disposed such that the temperature distribution in the reactor shows large thermal gradients of at least 20°C/cm, preferably 100°C/cm, thereby generating hot spots throughout the reactor. The temperature gradients can be controlled by controlling either the space velocity of the inlet reactant gases and/or the flow rate of the cooling medium.

This arrangement provides a combination of hot spots and colder zones within a small volume what allows molecules to reach a high kinetic energy while maintaining the reaction equilibrium on the side of the products. Thus, a conversion higher than that achievable under near-isothermal conditions is reached.

In order to maximize the reaction conversion, the minimal distance between the tubes should not be less than 1.5 times the tube diameter, or even better 2.0 times the tube diameter or more.

We will now describe the thermal management of the methanation reactor of the present invention. The thermal management of the methanation reactor has to address two issues: first, the reactor has to be pre-heated in order to start the reaction. Second, and as already mentioned, the methanation reaction is highly exothermic. Therefore, an adequate thermal management system has to be developed in order to effectively remove the heat from the reaction zone and to control the temperature of the chemical reactor.

As explained below, with this reactor, a conversion greater than 95% and even greater than 99% in a single stage reactor has been experimentally measured.

The chemical reactor of the present invention can be used for any exothermic reaction that is thermally limited. It is also particularly adapted for the production of a mixture of water and methane from carbon dioxide and hydrogen (Sabatier reaction). In any case, the application of this reactor is independent on the upstream (production of hydrogen and carbon dioxide) and downstream (use of the methane and water) processes.

**Example**

An example of the reactor and of its use will now be described.

In the present case, the target production was set to 100 gCH4/hr. The reactor was fed with a stoichiometric mixture of H2 and CO2, with nominal volumetric flows of 9.33 Nl/min and 2.33 Nl/min, respectively. The total power of the reactor amounted to around 2 kW, split in 1.54 kW contained in the produced methane and 0.44 kW supplied in the form of heat.

As a catalyst, a commercial ruthenium based catalyst was selected for the chemical reactor. The catalyst was supplied by Sigma Aldrich and has a 0.5% wt ruthenium loading on alumina. It is pressed in cylindrical pellets with an average diameter of 3.2mm and a length of 5mm. The reactor bed is filled with 250g of this catalyst. Transmission electron micrograph analysis (TEM, FEI, Tecnai G2 spirit twin) was conducted in order to determine the particle size of ruthenium. Both new and used catalyst was measured, with no significant change in the structure and particle size. The sample was prepared by mixing 0.01 g of sample in 1 mL ethanol for 30 min sonication and dispersing in carbon grid. The typical ruthenium particle size is in the range of 9 - 17 nm and the average size is 11.5 nm. This is slightly larger than reported by Kwak et al., who performed STEM images of a similar catalyst (0.1%wt Ru on $Al_2O_3$) and found ruthenium particle sizes of up to 5 nm once the catalyst had been used [27]. The average ruthenium particle size of 11.5 nm corresponds to a specific surface area of 22.4 m2/g when considering spherical particles.

As an adequate thermal management system, a heating collar with a power of 540W and a maximal allowable temperature of 400°C was installed around the reactor tube in order to preheat the reaction zone at startup. Further, the outer wall of the reactor tube was insulated such that the startup time is minimized.

The cooling of the reactor during operation was ensured by six cooling tubes embedded directly in the catalyst bed. The total heat generated in the chemical reactor was calculated following equation (3) below.

$$\dot{Q}\mathrm{jB\backslash} = \dot{n}\mathrm{HIJ}\ \Delta H. \qquad (3)$$

Thereby, the value for the reaction enthalpy had to be calculated at the reaction conditions.

It was assumed that the reaction occurs above 140°C and that water exits the reactor in the vapor phase. Typically, the water condensates outside of the reactor. Therefore, the value for the reaction enthalpy under operating conditions was calculated to 152 kJ/mol and the total heating power of the reaction amounts to 264W.

The maximal allowable thermal resistance given the existing thermal gradient between the reactor and the cooling fluid was computed and it came out that. Dissipating such a high heat load by using air as the cooling medium required a fluid velocity of over 100 m/s in the cooling tubes, which is not realistic. Therefore, water was chosen as the cooling medium. A minimal water flow rate of 1.1 g/s was required to fulfill the thermal requirements.

Regarding the setup, the layout of the experimental setup is represented graphically in Figure 6. The gas stream of the reactants was controlled by two mass flow meters (Vögtlin Red---y Smart). A third gas line was built in for helium or nitrogen, which can be used to flush the system or for calibration purposes. The reactor was designed to operate at temperatures up to 400°C and pressures up to 10 bar with a structural safety factor of 4. Pressure relief valves were installed on the main gas line and on the cooling loop to protect the system from overpressure. The flow rate of the cooling water was controlled by a proportional valve (Omega FSV 12). The exhaust stream of the reactor was cooled down in order to condensate the water. The composition of the remaining gases was analyzed in a Fourier Transformed Infrared Spectrometer (FT---IR, Alpha Bruker). Several pressure and temperature sensors were embedded in the system to accurately monitor the testing conditions.

The reactor performance was tested under various conditions of temperature and space velocity. For all experiments, the pressure was set to 5 bar. From Figure 1, it is seen that an increase in pressure past 5 bar only leads to a marginal increase in the equilibrium conversion. Further, and from the point of view of implementation, CO2 from atmospheric capture is available at low pressure. Thus, in order to avoid the use of an external compressor for the CO2, the reactor pressure is limited to 5 bar. The domain of investigation for the space velocity and the temperature was set to 0.14 - 0.55 s-1 and 140 - 400°C. These boundaries were defined to match the SSDS boundary conditions (quantity of methane produced), reactor limits (maximal temperature) as well as thermodynamic modelling and literature review. A total of 57 experiments were carried out in that domain. The calculated conversion are shown in Figure 4.

The temperature shown as reactor temperature is only a representative value measured at a single point in the catalyst bed, as shown in Figure 3 (left). In practice, large temperature gradients are present in the reactor because of the local chemical reaction releasing heat and the cooling tubes going directly through the catalyst bed. It was indeed observed that a temperature difference of almost 100°C exists within the catalyst. In the actual reactor, this behavior is expected to be even exacerbated due to inhomogeneous heat generation due to the spatial variation of the chemical reaction rate within the reactor.

The reactor of the present invention proved to reach a very high conversion of up to 99% at a temperature set point of 260°C, a pressure of 5 bar and a space velocity of 0.14 s-1. A CO2 conversion of 97% was reached at the target flow rate of 50 g/hr H2 at a temperature set point of 280°C. It was found that the process is limited by the kinetics rather than the thermodynamic equilibrium under the tested conditions. The obtained values are very close to - and in some cases even beyond - the theoretical thermodynamic equilibrium. This is explained by the inhomogeneous temperature distribution in the chemical reactor: while the temperature of the catalyst bed is measured at a single point, there is a temperature distribution within the catalyst bed enabling to balance both kinetics and equilibrium aspects. Finally, no degradation of the catalyst was observed after a period of four months of usage.

While the embodiments have been described in conjunction with a number of embodiments, it is evident that many alternatives, modifications and variations would be or are apparent to those of ordinary skill in the applicable arts. Accordingly, this disclosure is intended to embrace all such alternatives, modifications, equivalents and variations that are within the scope of this disclosure. This for example particularly the case regarding the different apparatuses which can be used.

## Claims

1. Chemical reactor comprising a catalyst bed enclosed in a reactor vessel and at least one cooling tube placed in the reactor vessel and passing through the catalyst bed, **characterized in that** the cooling tubes are disposed within the reactor so as to generate thermal gradients of at least 20°C/cm thereby generating hot spots throughout the reactor upon carrying out a reaction.

2. Chemical reactor according to claim 1, **characterized in that** the catalysts comprises at least one of Nickel, Cobalt and Ruthenium based catalysts.

3. Chemical reactor to claim 1 or 2, **characterized in that** the catalysts comprises 20% wt. $Ni/Al_2O_3$ or 3% wt. $Ru/Al_2O_3$.

4. Chemical reactor according to any one of claims 1 to 3, **characterized in that** it comprises at least two cooling tubes.

5. Chemical reactor according to any one of claims 1 to 8, **characterized in that** the minimal distance between the tubes is not less than 1.5 times the tube diameter.

6. Chemical reactor according to any one of claims 1 to 8, **characterized in that** the minimal distance between the tubes is not less than 2 times the tube diameter.

7. Chemical reactor according to any one of claims 1 to 4, **characterized in that** the temperature of the cooling medium is different in the different tubes.

8. Chemical reactor according to any one of claims 1 to 5, **characterized in that** the tubes are fed with a cooling medium such as water, oil or any other fluid suitable for this purpose.

9. Chemical reactor according to any one of claims 1 to 6, **characterized in that** the thermal gradients is at least 100°C/cm

10. Chemical reactor according to any one of claims 1 to 7, **characterized in that** the temperature gradients are controlled by controlling either the space velocity of the inlet reactant gases and/or the flow rate of the cooling medium.

**11.** Chemical reactor according to claim 11, **characterized in that** it further comprises a thermal management system adapted to remove the heat from the reaction zone and to control the temperature of the chemical reactor.

**12.** Chemical reactor according to any one of claims 1 to 9, **characterized in that** the reactor is adapted for exothermic chemical reaction.

**13.** Chemical reactor according to claim 10, **characterized in that** it is a methanation reactor.

Figure 1

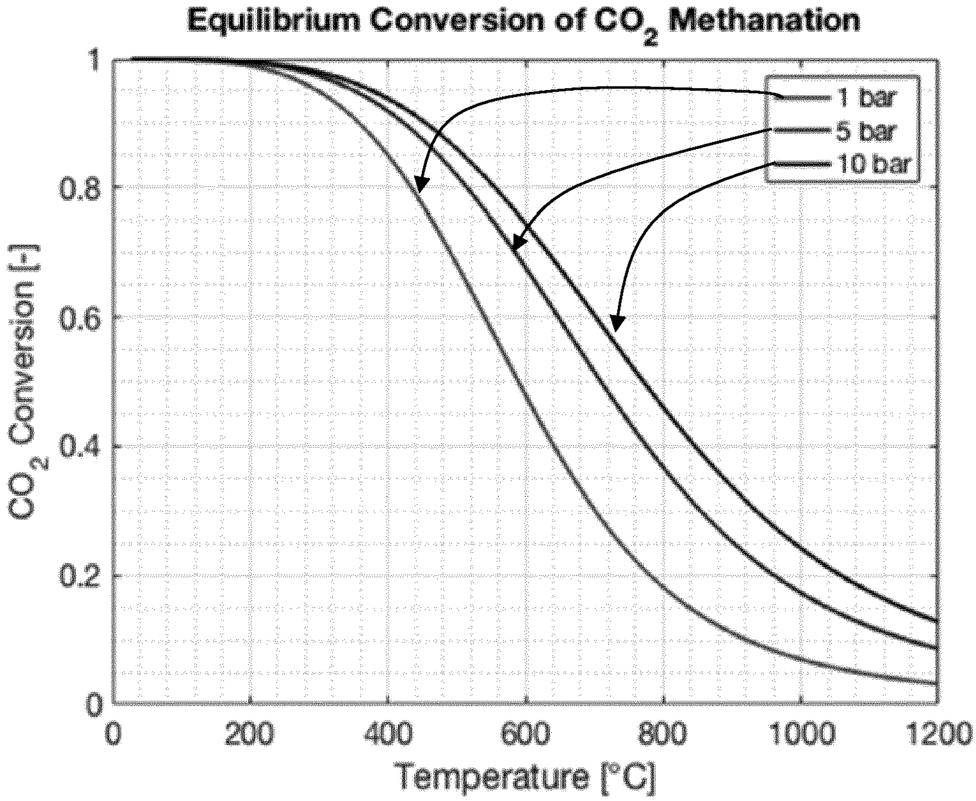


Figure 2

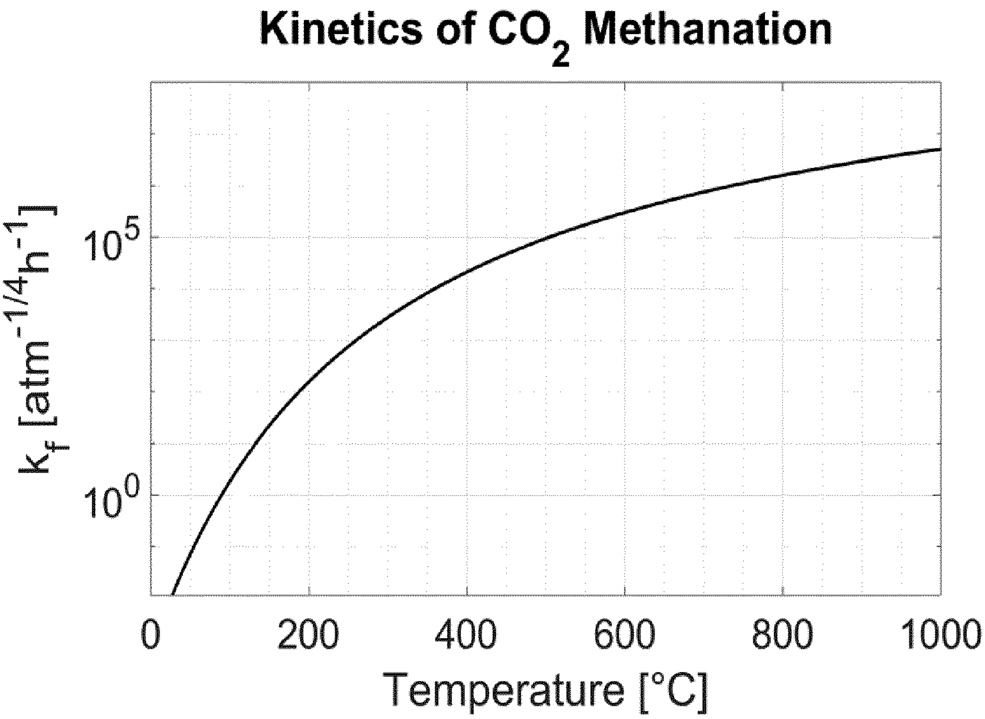

Figure 3

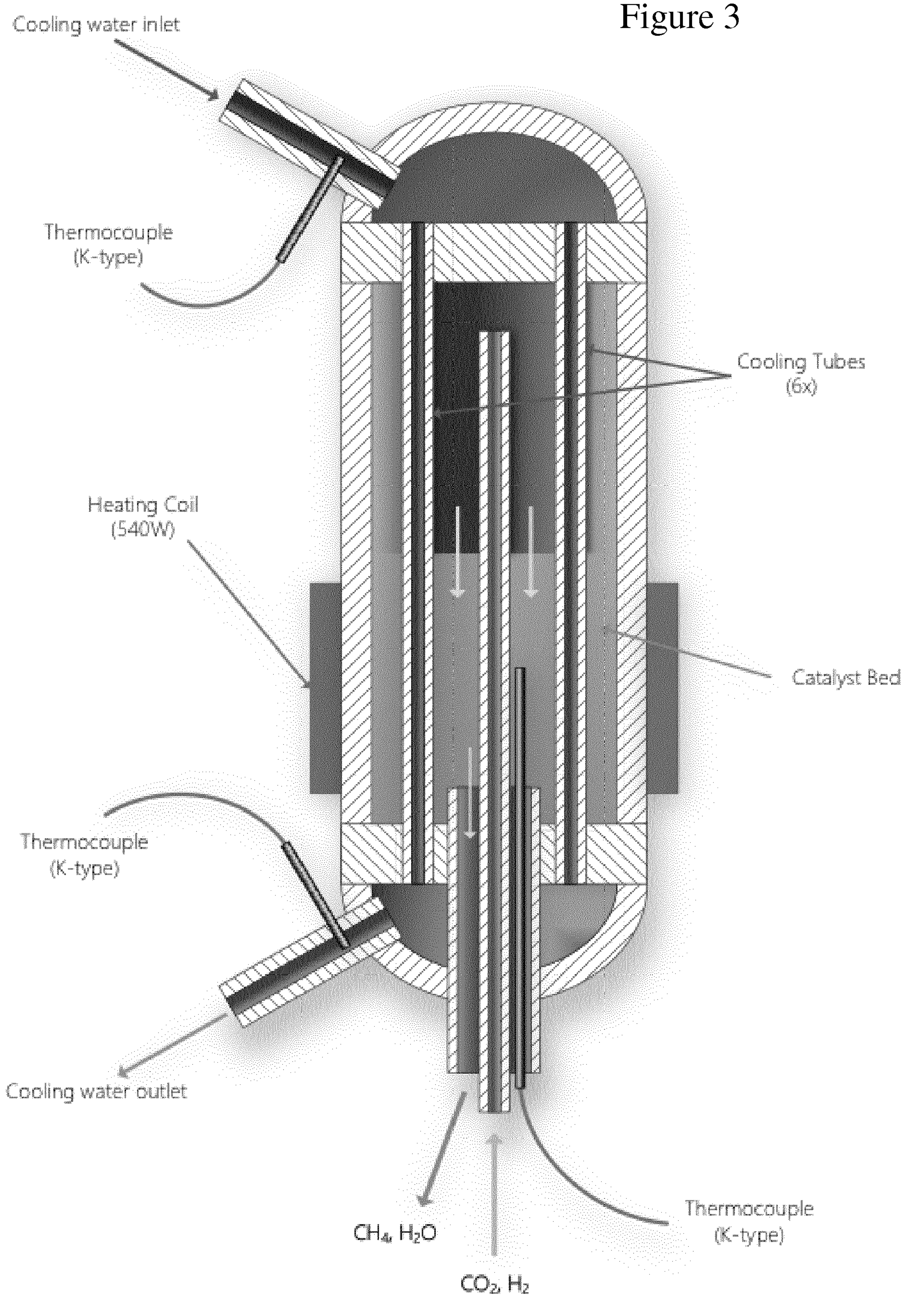
Cooling water inlet
Thermocouple (K-type)
Cooling Tubes (6x)
Heating Coil (540W)
Catalyst Bed
Thermocouple (K-type)
Cooling water outlet
$CH_4$, $H_2O$
$CO_2$, $H_2$
Thermocouple (K-type)

Figure 4

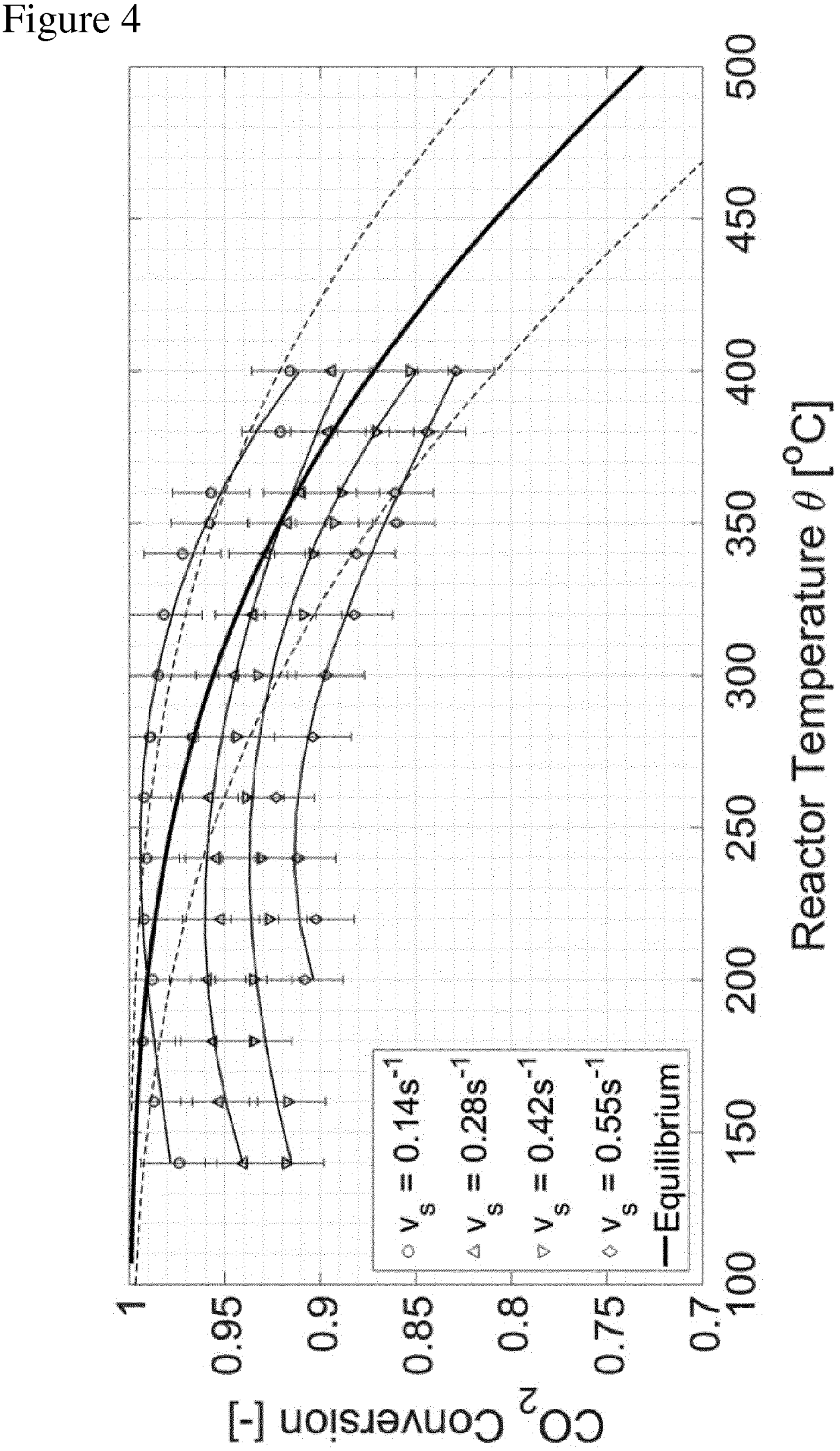

CO2 Conversion [-]
Reactor Temperature θ [°C]
vs = 0.14s-1
vs = 0.28s-1
vs = 0.42s-1
vs = 0.55s-1
Equilibrium
1
0.95
0.9
0.85
0.8
0.75
0.7
100
150
200
250
300
350
400
450
500

Figure 5

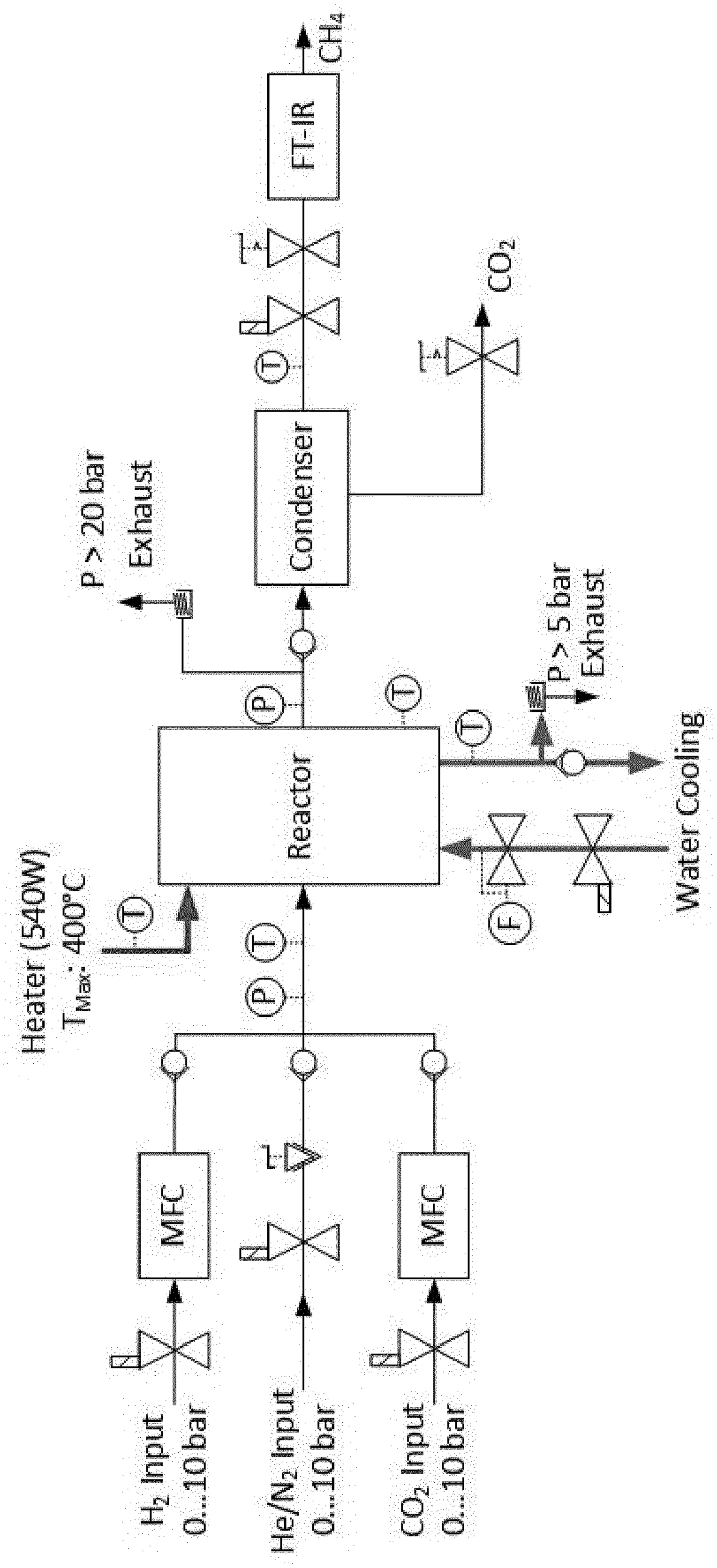

$H_2$ Input
0...10 bar
He/$N_2$ Input
0...10 bar
$CO_2$ Input
0...10 bar
MFC
MFC
Heater (540W)
$T_{Max}$: 400°C
Reactor
Water Cooling
P > 5 bar
Exhaust
P > 20 bar
Exhaust
Condenser
FT-IR
$CH_4$
$CO_2$

Figure 6

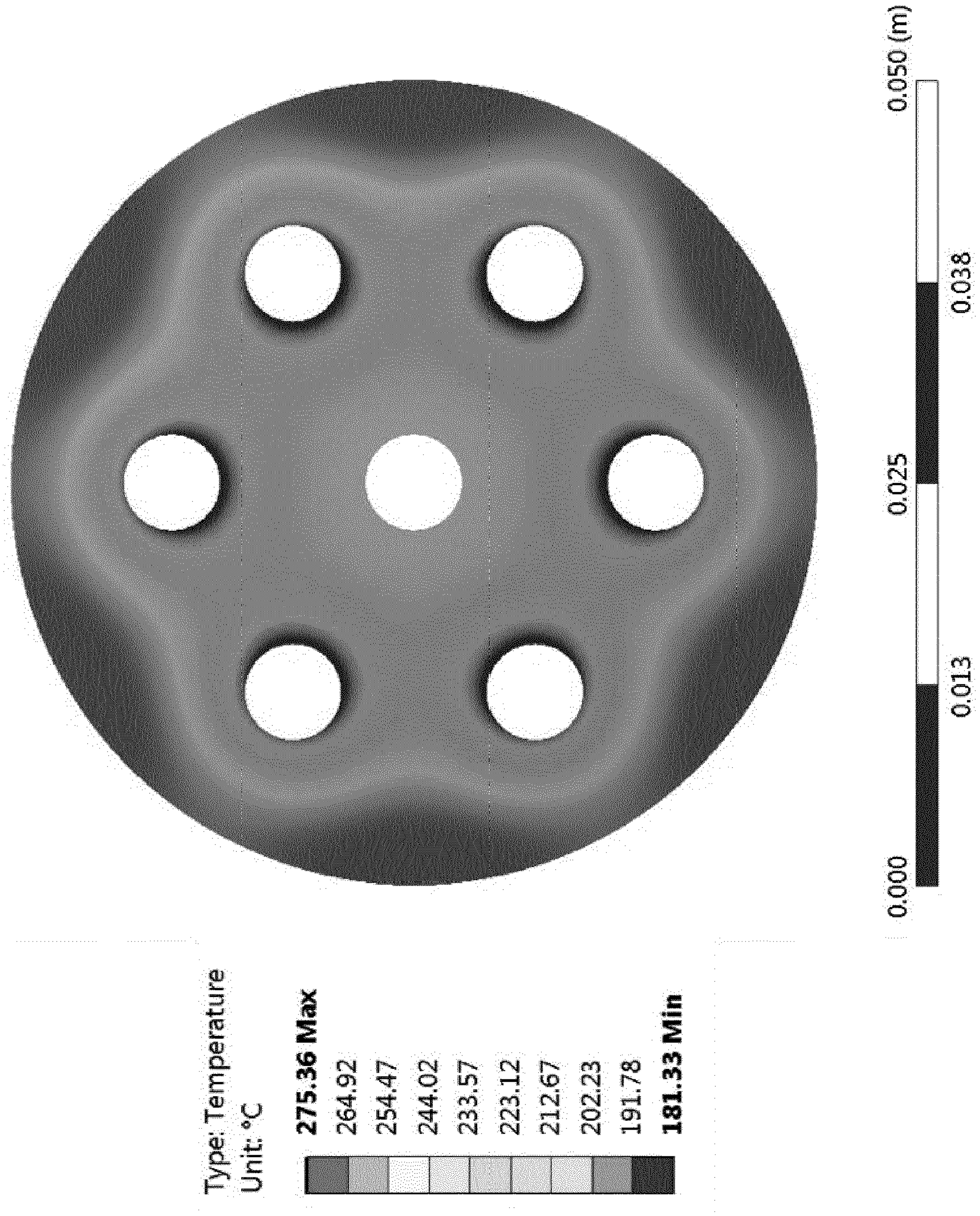
Type: Temperature
Unit: °C
275.36 Max
264.92
254.47
244.02
233.57
223.12
212.67
202.23
191.78
181.33 Min
0.000
0.013
0.025
0.038
0.050 (m)

Europäisches Patentamt
European Patent Office
Office européen des brevets

# EUROPEAN SEARCH REPORT

Application Number
EP 18 19 7885

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | NORIS GALLANDAT ET AL: "Experimental performance investigation of a 2 kW methanation reactor", SUSTAINABLE ENERGY & FUELS, vol. 2, no. 5, 1 January 2018 (2018-01-01), pages 1101-1110, XP055573623, ISSN: 2398-4902, DOI: 10.1039/C8SE00073E * the whole document * ----- | 1-13 | INV. B01J8/02 B01J8/00 B01J21/04 B01J23/46 B01J23/75 B01J23/755 C10L3/08 C07C1/12 |

TECHNICAL FIELDS SEARCHED (IPC)

B01J
C07C
C10L

The present search report has been drawn up for all claims

1

| Place of search | Date of completion of the search | Examiner |
|---|---|---|
| Munich | 25 March 2019 | Borello, Ettore |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- DE 102014010055 A1
- DE 102014011274